# EUROPEAN PATENT APPLICATION

(11) **EP 3 974 440 A1**
(43) Date of publication of application: **30.03.2022**
(21) Application number: 20197824.4
(22) Date of filing: 23.09.2020
(51) Int. Cl.: C07K 7/00, C07K 14/00, G01N 33/557, G01N 33/68

(54) **HIGH AFFINITY BINDERS OF PROTEINS OF THE ATG8 FAMILY**

(71) Applicant: UNIVERSITE PAUL SABATIER TOULOUSE III, 31062 Toulouse Cedex 9 (FR); Centre national de la recherche scientifique, 75016 Paris (FR)
(72) Inventor: RODRIGUEZ, Manuel S., 31400 Toulouse (FR); QUINET, Grégoire, 33200 Bordeaux (FR); LEGOUIS, Renaud, 28230 Epernon (FR)
(74) Representative: Germain Maureau

(57) **Abstract**

The present invention relates to (poly)peptides comprising a sequence consisting of a LC3-interacting region (LIR) core motif and specific linker peptidic sequences. These (poly)peptides selectively bind, with high affinity, protein(s) of the ATG8 family, and can advantageously be used for the capture and/or the detection of protein(s) of the ATG8 family, as well as the identification of autophagy inhibitors.

SEQUENCE LISTING

<110>  UNIVERSITE PAUL SABATIER
       CNRS

<120>  HIGH AFFINITY BINDERS OF PROTEINS OF THE ATG8 FAMILY

<130>  BR110613/LTI/CCU

<160>  37

<170>  PatentIn version 3.5

<210>  1
<211>  4
<212>  PRT
<213>  Artificial Sequence

<220>
<223>  Artificial sequence


<220>
<221>  MISC_FEATURE
<222>  (1)..(1)
<223>  Xaa is F, W or Y

<220>
<221>  MISC_FEATURE
<222>  (2)..(2)
<223>  Xaa can be any aminoacid

<220>
<221>  MISC_FEATURE
<222>  (3)..(3)
<223>  Xaa can be any aminoacid

<220>
<221>  MISC_FEATURE
<222>  (4)..(4)
<223>  Xaa is I, L or V

<400>  1

Xaa Xaa Xaa Xaa
1


<210>  2
<211>  4
<212>  PRT
<213>  Artificial Sequence

<220>
<223>  LIR core motif "F"

<400>  2

Phe Val Ile Ile
1


<210>  3

<211>  4
<212>  PRT
<213>  Artificial Sequence

<220>
<223>  LIR core motif "W"

<400>  3

Trp Glu Glu Leu
1


<210>  4
<211>  4
<212>  PRT
<213>  Artificial Sequence

<220>
<223>  LIR core motif "Y"

<400>  4

Tyr Asp Val Ile
1


<210>  5
<211>  12
<212>  PRT
<213>  Artificial Sequence

<220>
<223>  pepLIR-F

<400>  5

Ser Cys Asp Thr Asp Asp Phe Val Ile Ile Pro His
1               5                   10


<210>  6
<211>  36
<212>  DNA
<213>  Artificial Sequence

<220>
<223>  pepLIR-F (NA)

<400>  6
agctgcgata cagatgactt tgtgattatt ccgcat                                36


<210>  7
<211>  12
<212>  PRT
<213>  Artificial Sequence

<220>
<223>  pepLIR-W

<400>  7

Glu Ser Leu Asp Asp Asp Trp Glu Glu Leu Asp Pro
1               5                   10

<210>  8
<211>  36
<212>  DNA
<213>  Artificial Sequence

<220>
<223>  PepLIR-W (NA)

<400>  8
gaatctctag atgacgattg ggaagaacta gaccct                                36

<210>  9
<211>  12
<212>  PRT
<213>  Artificial Sequence

<220>
<223>  PepLIR-Y

<400>  9

Asp Ala Ala Ser Arg Asp Tyr Asp Val Ile Leu Glu
1               5                   10

<210>  10
<211>  36
<212>  DNA
<213>  Artificial Sequence

<220>
<223>  PepLIR-Y (NA)

<400>  10
gatgcagctt ctagagatta tgatgtgatt ctggaa                                36

<210>  11
<211>  6
<212>  PRT
<213>  Artificial Sequence

<220>
<223>  N-linker F

<400>  11

Ser Cys Asp Thr Asp Asp
1               5

<210>  12
<211>  6
<212>  PRT
<213>  Artificial Sequence

<220>
<223>  N-linker W

<400>  12

Glu Ser Leu Asp Asp Asp
1               5


<210>  13
<211>  6
<212>  PRT
<213>  Artificial Sequence

<220>
<223>  N-linker Y

<400>  13

Asp Ala Ala Ser Arg Asp
1               5


<210>  14
<211>  24
<212>  PRT
<213>  Artificial Sequence

<220>
<223>  PepLIR2-F

<400>  14

Ser Cys Asp Thr Asp Asp Phe Val Ile Ile Pro His Ser Cys Asp Thr
1               5                   10                  15


Asp Asp Phe Val Ile Ile Pro His
            20


<210>  15
<211>  72
<212>  DNA
<213>  Artificial Sequence

<220>
<223>  PepLIR2-F (NA)

<400>  15
agctgcgata cagatgactt tgtgattatt ccgcatagct gcgatacaga tgactttgtg      60

attattccgc at                                                          72


<210>  16
<211>  24
<212>  PRT
<213>  Artificial Sequence

<220>
<223>  PepLIR2-W

<400>  16

Glu Ser Leu Asp Asp Asp Trp Glu Glu Leu Asp Pro Glu Ser Leu Asp
1               5                   10                  15

Asp Asp Trp Glu Glu Leu Asp Pro
            20

<210>  17
<211>  72
<212>  DNA
<213>  Artificial Sequence

<220>
<223>  PepLIR2-W (NA)

<400>  17
gaatctctag atgacgattg ggaagaacta gaccctgaat ctctagatga cgattgggaa      60

gaactagacc ct                                                          72

<210>  18
<211>  24
<212>  PRT
<213>  Artificial Sequence

<220>
<223>  PepLIR2-Y

<400>  18

Asp Ala Ala Ser Arg Asp Tyr Asp Val Ile Leu Glu Asp Ala Ala Ser
1               5                   10                  15

Arg Asp Tyr Asp Val Ile Leu Glu
            20

<210>  19
<211>  72
<212>  DNA
<213>  Artificial Sequence

<220>
<223>  PepLIR2-Y (NA)

<400>  19
gatgcagctt ctagagatta tgatgtgatt ctggaagatg cagcttctag agattatgat      60

gtgattctgg aa                                                          72

<210>  20
<211>  288
<212>  PRT
<213>  Artificial Sequence

<220>
<223>  LIR-trap Fx2

<400>  20

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His His His His His His Gly Ser Ser
225                 230                 235                 240

Cys Asp Thr Asp Asp Phe Val Ile Ile Pro His Ser Cys Asp Thr Asp
                245                 250                 255

Asp Phe Val Ile Ile Pro His Gly Ser Pro Glu Phe Pro Gly Val Asp
            260                 265                 270

Leu Glu Gly Lys Pro Ile Pro Asn Pro Leu Leu Gly Leu Glu Ser Thr
        275                 280                 285

<210>  21
<211>  908
<212>  DNA
<213>  Artificial Sequence

<220>
<223>  LIR-trap Fx2 (NA)

<400>  21
atgtccccta tactaggtta ttggaaaatt aagggccttg tgcaacccac tcgacttctt      60

ttggaatatc ttgaagaaaa atatgaagag catttgtatg agcgcgatga aggtgataaa     120

tggcgaaaca aaaagtttga attgggtttg gagtttccca atcttcctta ttatattgat     180

ggtgatgtta aattaacaca gtctatggcc atcatacgtt atatagctga caagcacaac     240

atgttgggtg gttgtccaaa agagcgtgca gagatttcaa tgcttgaagg agcggttttg     300

gatattagat acggtgtttc gagaattgca tatagtaaag actttgaaac tctcaaagtt     360

gattttctta gcaagctacc tgaaatgctg aaaatgttcg aagatcgttt atgtcataaa     420

acatatttaa atggtgatca tgtaacccat cctgacttca tgttgtatga cgctcttgat     480

gttgttttat acatggaccc aatgtgcctg gatgcgttcc caaaattagt ttgttttaaa     540

aaacgtattg aagctatccc acaaattgat aagtacttga aatccagcaa gtatatagca     600

tggcctttgc agggctggca agccacgttt ggtggtggcg accatcctcc aaaatcggat     660

ctggaagttc tgttccaggg gcccctggga tctcatcacc atcaccatca cggatctagc     720

tgcgatacag atgactttgt gattattccg catagctgcg atacagatga ctttgtgatt     780

attccgcatg gatccccgga attccccggg gtcgacctcg agggaaagcc aatacctaat     840

ccactacttg gactagaatc cacataggcg gccgcatcgt gactgactga cgatctgcct     900

cgcgcgtt                                                              908

<210>  22
<211>  314
<212>  PRT
<213>  Artificial Sequence

<220>
<223>  LIR-trap Fx4

<400>  22

Met Ser Pro Ile Leu Gly Tyr Trp Lys Ile Lys Gly Leu Val Gln Pro

1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His His His His His His Gly Ser Ser
225                 230                 235                 240

Cys Asp Thr Asp Asp Phe Val Ile Ile Pro His Ser Cys Asp Thr Asp
                245                 250                 255

Asp Phe Val Ile Ile Pro His Gly Ser Ser Cys Asp Thr Asp Asp Phe
            260                 265                 270

Val Ile Ile Pro His Ser Cys Asp Thr Asp Asp Phe Val Ile Ile Pro
        275                 280                 285

His Gly Ser Pro Glu Phe Pro Gly Val Asp Leu Glu Gly Lys Pro Ile
    290                 295                 300

Pro Asn Pro Leu Leu Gly Leu Glu Ser Thr
305                 310

<210>  23
<211>  975
<212>  DNA
<213>  Artificial Sequence

<220>
<223>  LIR-trap Fx4 (NA)

<400>  23
atgtccccta tactaggtta ttggaaaatt aagggccttg tgcaacccac tcgacttctt      60

ttggaatatc ttgaagaaaa atatgaagag catttgtatg agcgcgatga aggtgataaa     120

tggcgaaaca aaaagtttga attgggtttg gagtttccca atcttcctta ttatattgat     180

ggtgatgtta aattaacaca gtctatggcc atcatacgtt atatagctga caagcacaac     240

atgttgggtg gttgtccaaa agagcgtgca gagatttcaa tgcttgaagg agcggttttg     300

gatattagat acggtgtttc gagaattgca tatagtaaag actttgaaac tctcaaagtt     360

gattttctta gcaagctacc tgaaatgctg aaaatgttcg aagatcgttt atgtcataaa     420

acatatttaa atggtgatca tgtaacccat cctgacttca tgttgtatga cgctcttgat     480

gttgttttat acatggaccc aatgtgcctg gatgcgttcc caaaattagt ttgttttaaa     540

aaacgtattg aagctatccc acaaattgat aagtacttga aatccagcaa gtatatagca     600

tggcctttgc agggctggca agccacgttt ggtggtggcg accatcctcc aaaatcggat     660

ctggaagttc tgttccaggg gcccctggga tctcatcacc atcaccatca cggatctagc     720

tgcgatacag atgactttgt gattattccg catagctgcg atacagatga ctttgtgatt     780

attccgcatg gatctagctg cgatacagat gactttgtga ttattccgca tagctgcgat     840

acagatgact ttgtgattat tccgcatgga tccccggaat tccccggggt cgacctcgag     900

ggaaagccaa tacctaatcc actacttgga ctagaatcca cataggcggc cgcatcgtga     960

ctgactgacg atctg                                                      975

<210>  24
<211>  366
<212>  PRT

<213>  Artificial Sequence

<220>
<223>  LIR-trap Fx8

<400>  24

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His His His His His His Gly Ser Ser
225                 230                 235                 240

Cys Asp Thr Asp Asp Phe Val Ile Ile Pro His Ser Cys Asp Thr Asp
                245                 250                 255

Asp Phe Val Ile Ile Pro His Gly Ser Ser Cys Asp Thr Asp Asp Phe
            260                 265                 270

Val Ile Ile Pro His Ser Cys Asp Thr Asp Asp Phe Val Ile Ile Pro
        275                 280                 285

His Gly Ser Ser Cys Asp Thr Asp Asp Phe Val Ile Ile Pro His Ser
    290                 295                 300

Cys Asp Thr Asp Asp Phe Val Ile Ile Pro His Gly Ser Ser Cys Asp
305                 310                 315                 320

Thr Asp Asp Phe Val Ile Ile Pro His Ser Cys Asp Thr Asp Asp Phe
                325                 330                 335

Val Ile Ile Pro His Gly Ser Pro Glu Phe Pro Gly Val Asp Leu Glu
            340                 345                 350

Gly Lys Pro Ile Pro Asn Pro Leu Leu Gly Leu Glu Ser Thr
        355                 360                 365

<210>  25
<211>  1002
<212>  DNA
<213>  Artificial Sequence

<220>
<223>  LIR-trap Fx8 (NA)

<400>  25
aaaaagtttg aattgggttt ggagtttccc aatcttcctt attatattga tggtgatgtt      60

aaattaacac agtctatggc catcatacgt tatatagctg acaagcacaa catgttgggt     120

ggttgtccaa aagagcgtgc agagatttca atgcttgaag gagcggtttt ggatattaga     180

tacggtgttt cgagaattgc atatagtaaa gactttgaaa ctctcaaagt tgattttctt     240

agcaagctac ctgaaatgct gaaaatgttc gaagatcgtt tatgtcataa aacatattta     300

aatggtgatc atgtaaccca tcctgacttc atgttgtatg acgctcttga tgttgtttta     360

tacatggacc caatgtgcct ggatgcgttc ccaaaattag tttgttttaa aaaacgtatt     420

gaagctatcc cacaaattga taagtacttg aaatccagca agtatatagc atggcctttg     480

cagggctggc aagccacgtt tggtggtggc gaccatcctc caaaatcgga tctggaagtt     540

ctgttccagg ggcccctggg atctcatcac catcaccatc acggatctag ctgcgataca 600

gatgactttg tgattattcc gcatagctgc gatacagatg actttgtgat tattccgcat 660

ggatctagct gcgatacaga tgactttgtg attattccgc atagctgcga tacagatgac 720

tttgtgatta ttccgcatgg atctagctgc gatacagatg actttgtgat tattccgcat 780

agctgcgata cagatgactt tgtgattatt ccgcatggat ctagctgcga tacagatgac 840

tttgtgatta ttccgcatag ctgcgataca gatgactttg tgattattcc gcatggatcc 900

ccggaattcc ccggggtcga cctcgaggga aagccaatac ctaatccact acttggacta 960

gaatccacat aggcggccgc atcgtgactg actgacgatc tg 1002

<210> 26
<211> 288
<212> PRT
<213> Artificial Sequence

<220>
<223> LIR-trap Wx2

<400> 26

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His His His His His His Gly Ser Glu
225                 230                 235                 240

Ser Leu Asp Asp Asp Trp Glu Glu Leu Asp Pro Glu Ser Leu Asp Asp
                245                 250                 255

Asp Trp Glu Glu Leu Asp Pro Gly Ser Pro Glu Phe Pro Gly Val Asp
            260                 265                 270

Leu Glu Gly Lys Pro Ile Pro Asn Pro Leu Leu Gly Leu Glu Ser Thr
        275                 280                 285

<210>  27
<211>  902
<212>  DNA
<213>  Artificial Sequence

<220>
<223>  LIR-trap Wx2 (NA)

<400>  27
atgtccccta tactaggtta ttggaaaatt aagggccttg tgcaacccac tcgacttctt      60

ttggaatatc ttgaagaaaa atatgaagag catttgtatg agcgcgatga aggtgataaa     120

tggcgaaaca aaaagtttga attgggtttg gagtttccca atcttcctta ttatattgat     180

ggtgatgtta aattaacaca gtctatggcc atcatacgtt atatagctga caagcacaac     240

atgttgggtg gttgtccaaa agagcgtgca gagatttcaa tgcttgaagg agcggttttg     300

gatattagat acggtgtttc gagaattgca tatagtaaag actttgaaac tctcaaagtt     360

gattttctta gcaagctacc tgaaatgctg aaaatgttcg aagatcgttt atgtcataaa     420

acatatttaa atggtgatca tgtaacccat cctgacttca tgttgtatga cgctcttgat     480

gttgttttat acatggaccc aatgtgcctg gatgcgttcc caaaattagt ttgttttaaa     540

aaacgtattg aagctatccc acaaattgat aagtacttga aatccagcaa gtatatagca 600

tggcctttgc agggctggca agccacgttt ggtggtggcg accatcctcc aaaatcggat 660

ctggaagttc tgttccaggg gcccctggga tctcatcacc atcaccatca cggatctgaa 720

tctctagatg acgattggga agaactagac cctgaatctc tagatgacga ttgggaagaa 780

ctagaccctg gatccccgga attccccggg gtcgacctcg agggaaagcc aatacctaat 840

ccactacttg gactagaatc cacataggcg gccgcatcgt gactgactga cgatctgcct 900

cg 902

<210> 28
<211> 314
<212> PRT
<213> Artificial Sequence

<220>
<223> LIR-trap Wx4

<400> 28

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His His His His His His Gly Ser Glu
225                 230                 235                 240

Ser Leu Asp Asp Asp Trp Glu Glu Leu Asp Pro Glu Ser Leu Asp Asp
                245                 250                 255

Asp Trp Glu Glu Leu Asp Pro Gly Ser Glu Ser Leu Asp Asp Asp Trp
            260                 265                 270

Glu Glu Leu Asp Pro Glu Ser Leu Asp Asp Asp Trp Glu Glu Leu Asp
        275                 280                 285

Pro Gly Ser Pro Glu Phe Pro Gly Val Asp Leu Glu Gly Lys Pro Ile
    290                 295                 300

Pro Asn Pro Leu Leu Gly Leu Glu Ser Thr
305                 310

<210>  29
<211>  987
<212>  DNA
<213>  Artificial Sequence

<220>
<223>  LIR-trap Wx4 (NA)

<400>  29
atgtccccta tactaggtta ttggaaaatt aagggccttg tgcaacccac tcgacttctt      60

ttggaatatc ttgaagaaaa atatgaagag catttgtatg agcgcgatga aggtgataaa     120

tggcgaaaca aaaagtttga attgggtttg gagtttccca atcttcctta ttatattgat     180

ggtgatgtta aattaacaca gtctatggcc atcatacgtt atatagctga caagcacaac     240

atgttgggtg gttgtccaaa agagcgtgca gagatttcaa tgcttgaagg agcggttttg     300

gatattagat acggtgtttc gagaattgca tatagtaaag actttgaaac tctcaaagtt     360

gattttctta gcaagctacc tgaaatgctg aaaatgttcg aagatcgttt atgtcataaa 420

acatatttaa atggtgatca tgtaacccat cctgacttca tgttgtatga cgctcttgat 480

gttgttttat acatggaccc aatgtgcctg gatgcgttcc caaaattagt ttgttttaaa 540

aaacgtattg aagctatccc acaaattgat aagtacttga aatccagcaa gtatatagca 600

tggcctttgc agggctggca agccacgttt ggtggtggcg accatcctcc aaaatcggat 660

ctggaagttc tgttccaggg gcccctggga tctcatcacc atcaccatca cggatctgaa 720

tctctagatg acgattggga agaactagac cctgaatctc tagatgacga ttgggaagaa 780

ctagaccctg gatctgaatc tctagatgac gattgggaag aactagaccc tgaatctcta 840

gatgacgatt gggaagaact agaccctgga tccccggaat tccccggggt cgacccgggg 900

gtcgacctcg agggaaagcc aatacctaat ccactacttg gactagaatc cacataggcg 960

gccgcatcgt gactgactga cgatctg 987

<210> 30
<211> 366
<212> PRT
<213> Artificial Sequence

<220>
<223> LIR-trap Wx8

<400> 30

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His His His His His His Gly Ser Glu
225                 230                 235                 240

Ser Leu Asp Asp Asp Trp Glu Glu Leu Asp Pro Glu Ser Leu Asp Asp
                245                 250                 255

Asp Trp Glu Glu Leu Asp Pro Gly Ser Glu Ser Leu Asp Asp Asp Trp
            260                 265                 270

Glu Glu Leu Asp Pro Glu Ser Leu Asp Asp Asp Trp Glu Glu Leu Asp
        275                 280                 285

Pro Gly Ser Glu Ser Leu Asp Asp Asp Trp Glu Glu Leu Asp Pro Glu
    290                 295                 300

Ser Leu Asp Asp Asp Trp Glu Glu Leu Asp Pro Gly Ser Glu Ser Leu
305                 310                 315                 320

Asp Asp Asp Trp Glu Glu Leu Asp Pro Glu Ser Leu Asp Asp Asp Trp
                325                 330                 335

Glu Glu Leu Asp Pro Gly Ser Pro Glu Phe Pro Gly Val Asp Leu Glu
            340                 345                 350

Gly Lys Pro Ile Pro Asn Pro Leu Leu Gly Leu Glu Ser Thr
        355                 360                 365

<210> 31

<211>  1143
<212>  DNA
<213>  Artificial Sequence

<220>
<223>  LIR-trap Wx8 (NA)

<400>  31
atgtccccta tactaggtta ttggaaaatt aagggccttg tgcaacccac tcgacttctt      60

ttggaatatc ttgaagaaaa atatgaagag catttgtatg agcgcgatga aggtgataaa     120

tggcgaaaca aaaagtttga attgggtttg gagtttccca atcttcctta ttatattgat     180

ggtgatgtta aattaacaca gtctatggcc atcatacgtt atatagctga caagcacaac     240

atgttgggtg gttgtccaaa agagcgtgca gagatttcaa tgcttgaagg agcggttttg     300

gatattagat acggtgtttc gagaattgca tatagtaaag actttgaaac tctcaaagtt     360

gattttctta gcaagctacc tgaaatgctg aaaatgttcg aagatcgttt atgtcataaa     420

acatatttaa atggtgatca tgtaacccat cctgacttca tgttgtatga cgctcttgat     480

gttgttttat acatggaccc aatgtgcctg gatgcgttcc caaaattagt ttgttttaaa     540

aaacgtattg aagctatccc acaaattgat aagtacttga aatccagcaa gtatatagca     600

tggcctttgc agggctggca agccacgttt ggtggtggcg accatcctcc aaaatcggat     660

ctggaagttc tgttccaggg gcccctggga tctcatcacc atcaccatca cggatctgaa     720

tctctagatg acgattggga agaactagac cctgaatctc tagatgacga ttgggaagaa     780

ctagaccctg gatctgaatc tctagatgac gattgggaag aactagaccc tgaatctcta     840

gatgacgatt gggaagaact agaccctgga tccgaatctc tagatgacga ttgggaagaa     900

ctagaccctg aatctctaga tgacgattgg gaagaactag accctggatc cgaatctcta     960

gatgacgatt gggaagaact agaccctgaa tctctagatg acgattggga agaactagac    1020

cctggatccc cggaattccc cggggtcgac cccggggtcg acctcgaggg aaagccaata    1080

cctaatccac tacttggact agaatccaca taggcggccg catcgtgact gactgacgat    1140

ctg                                                                  1143


<210>  32
<211>  288
<212>  PRT
<213>  Artificial Sequence

<220>
<223>  LIR-trap Yx2

<400>  32

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His His His His His His Gly Ser Asp
225                 230                 235                 240

Ala Ala Ser Arg Asp Tyr Asp Val Ile Leu Glu Asp Ala Ala Ser Arg
                245                 250                 255

Asp Tyr Asp Val Ile Leu Glu Gly Ser Pro Glu Phe Pro Gly Val Asp
            260                 265                 270

Leu Glu Gly Lys Pro Ile Pro Asn Pro Leu Leu Gly Leu Glu Ser Thr
        275                 280                 285

<210>  33
<211>  905
<212>  DNA
<213>  Artificial Sequence

<220>
<223>  LIR-trap Yx2 (NA)

<400>  33
atgtccccta tactaggtta ttggaaaatt aagggccttg tgcaacccac tcgacttctt        60

ttggaatatc ttgaagaaaa atatgaagag catttgtatg agcgcgatga aggtgataaa       120

tggcgaaaca aaaagtttga attgggtttg gagtttccca atcttcctta ttatattgat       180

ggtgatgtta aattaacaca gtctatggcc atcatacgtt atatagctga caagcacaac       240

atgttgggtg gttgtccaaa agagcgtgca gagatttcaa tgcttgaagg agcggttttg       300

gatattagat acggtgtttc gagaattgca tatagtaaag actttgaaac tctcaaagtt       360

gattttctta gcaagctacc tgaaatgctg aaaatgttcg aagatcgttt atgtcataaa       420

acatatttaa atggtgatca tgtaacccat cctgacttca tgttgtatga cgctcttgat       480

gttgttttat acatggaccc aatgtgcctg gatgcgttcc caaaattagt ttgttttaaa       540

aaacgtattg aagctatccc acaaattgat aagtacttga aatccagcaa gtatatagca       600

tggcctttgc agggctggca agccacgttt ggtggtggcg accatcctcc aaaatcggat       660

ctggaagttc tgttccaggg gcccctggga tctcatcacc atcaccatca cggatctgat       720

gcagcttcta gagattatga tgtgattctg gaagatgcag cttctagaga ttatgatgtg       780

attctggaag gatccccgga attccccggg gtcgacctcg agggaaagcc aatacctaat       840

ccactacttg gactagaatc cacataggcg gccgcatcgt gactgactga cgatctgcct       900

cgcgc                                                                   905

<210>  34
<211>  314
<212>  PRT
<213>  Artificial Sequence

<220>
<223>  LIR-trap Yx4

<400>  34

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His His His His His His Gly Ser Asp
225                 230                 235                 240

Ala Ala Ser Arg Asp Tyr Asp Val Ile Leu Glu Asp Ala Ala Ser Arg
                245                 250                 255

Asp Tyr Asp Val Ile Leu Glu Gly Ser Asp Ala Ala Ser Arg Asp Tyr
            260                 265                 270

Asp Val Ile Leu Glu Asp Ala Ala Ser Arg Asp Tyr Asp Val Ile Leu

275                 280                 285

Glu Gly Ser Pro Glu Phe Pro Gly Val Asp Leu Glu Gly Lys Pro Ile
    290                 295                 300

Pro Asn Pro Leu Leu Gly Leu Glu Ser Thr
305                 310

<210>  35
<211>  983
<212>  DNA
<213>  Artificial Sequence

<220>
<223>  LIR-trap Yx4 (NA)

<400>  35
gtcccctata ctaggttatt gaaaattaag ggccttgtgc aacccactcg acttcttttg      60

gaatatcttg aagaaaaata tgaagagcat ttgtatgagc gcgatgaagg tgataaatgg     120

cgaaacaaaa agtttgaatt gggtttggag tttcccaatc ttccttatta tattgatggt     180

gatgttaaat taacacagtc tatggccatc atacgttata tagctgacaa gcacaacatg     240

ttgggtggtt gtccaaaaga gcgtgcagag atttcaatgc ttgaaggagc ggttttggat     300

attagatacg gtgtttcgag aattgcatat agtaaagact ttgaaactct caaagttgat     360

tttcttagca agctacctga aatgctgaaa atgttcgaag atcgtttatg tcataaaaca     420

tatttaaatg gtgatcatgt aacccatcct gacttcatgt tgtatgacgc tcttgatgtt     480

gttttataca tggacccaat gtgcctggat gcgttcccaa aattagtttg ttttaaaaaa     540

cgtattgaag ctatcccaca aattgataag tacttgaaat ccagcaagta tatagcatgg     600

cctttgcagg gctggcaagc cacgtttggt ggtggcgacc atcctccaaa atcggatctg     660

gaagttctgt tccaggggcc cctgggatct catcaccatc accatcacgg atctgatgca     720

gcttctagag attatgatgt gattctggaa gatgcagctt ctagagatta tgatgtgatt     780

ctggaaggat ctgatgcagc ttctagagat tatgatgtga ttctggaaga tgcagcttct     840

agagattatg atgtgattct ggaaggatcc ccggaattcc ccggggtcga cctcgaggga     900

aagccaatac ctaatccact acttggacta gaatccacat aggcggccgc atcgtgactg     960

actgacgatc tgcctcgcgc gtt                                             983

<210>  36
<211>  366
<212>  PRT
<213>  Artificial Sequence

<220>
<223>  LIR-trap Yx8

<400>  36

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His His His His His His Gly Ser Asp
225                 230                 235                 240

Ala Ala Ser Arg Asp Tyr Asp Val Ile Leu Glu Asp Ala Ala Ser Arg

245                 250                 255

Asp Tyr Asp Val Ile Leu Glu Gly Ser Asp Ala Ala Ser Arg Asp Tyr
            260                 265                 270

Asp Val Ile Leu Glu Asp Ala Ala Ser Arg Asp Tyr Asp Val Ile Leu
        275                 280                 285

Glu Gly Ser Asp Ala Ala Ser Arg Asp Tyr Asp Val Ile Leu Glu Asp
    290                 295                 300

Ala Ala Ser Arg Asp Tyr Asp Val Ile Leu Glu Gly Ser Asp Ala Ala
305                 310                 315                 320

Ser Arg Asp Tyr Asp Val Ile Leu Glu Asp Ala Ala Ser Arg Asp Tyr
                325                 330                 335

Asp Val Ile Leu Glu Gly Ser Pro Glu Phe Pro Gly Val Asp Leu Glu
            340                 345                 350

Gly Lys Pro Ile Pro Asn Pro Leu Leu Gly Leu Glu Ser Thr
        355                 360                 365

<210>  37
<211>  568
<212>  DNA
<213>  Artificial Sequence

<220>
<223>  LIR-trap Yx8 (NA)

<400>  37
aaatcggatc tggaagttct gttccagggg cccctgggat ctcatcacca tcaccatcac      60

ggatctgatg cagcttctag agattatgat gtgattctgg aagatgcagc ttctagagat     120

tatgatgtga ttctggaagg atctgatgca gcttctagag attatgatgt gattctggaa     180

gatgcagctt ctagagatta tgatgtgatt ctggaaggat ctgatgcagc ttctagagat     240

tatgatgtga ttctggaaga tgcagcttct agagattatg atgtgattct ggaaggatct     300

gatgcagctt ctagagatta tgatgtgatt ctggaagatg cagcttctag agattatgat     360

gtgattctgg aaggatcccc ggaattcccc ggggtcgacc tcgagggaaa gccaatacct     420

aatccactac ttggactaga atccacatag gcggccgcat cgtgactgac tgacgatctg     480

cctcgcgcgt ttcggtgatg acggtgaaaa cctctgacac atgcagctcc cggagacggt     540

cacagcttgt ctgtaagcgg atgccggg                                        568

## Description

### FIELD OF THE INVENTION

The present invention relates to (poly)peptides comprising a sequence consisting of a LC3-interacting region (LIR) core motif and specific linker peptidic sequences. These (poly)peptides selectively bind, with high affinity, protein(s) of the ATG8 family, and can advantageously be used for the capture and/or the detection of protein(s) of the ATG8 family, as well as the identification of autophagy inhibitors.

### BACKGROUND OF THE INVENTION

Autophagy is an essential intracellular pathway, which ensures the degradation of large cargos, such as organelles (e.g. damaged mitochondria), bulk proteins or large protein complexes, thus playing an important role in protein homeostasis (or 'proteostasis') within the cell, and maintaining the stability of the cellular proteome and the organism itself. Although basal levels of autophagy run under normal conditions, stresses such as starvation, infections, heat or drug treatment modulate autophagy activation. Dysregulation of autophagy is involved in a number of pathologies, such as cancers, neurodegenerative diseases (e.g. Parkinson's and Alzheimer's diseases), infections (bacterial or viral) and inflammatory diseases.

Autophagy is a highly conserved process, which exists in mammals, plants, insects, yeasts and fungi. The degradation process requires mammalian lysosomes or plant/yeast vacuoles to fuse with large cup-shaped double-membraned organelles, isolated and elongated from intracellular membranes sources and enclosing a portion of cytoplasm, called autophagosomes, in which substrates have been engulfed. The autolysosome hence formed drives substrates degradation by hydrolases. Understanding how this proteolytic pathway is regulated is crucial to understand cellular physiology and pathology, but also for the identification of biomarkers as well as drug targets that could be used to develop more specific and successful treatments. However, despite considerable efforts, current methods to study and monitor autophagy (as reviewed in Yoshii and Mizushima, Int. J. Mol. Sci. (2017), 18:1865) are often complicated and the results can sometimes be misinterpreted.

Although early studies supported the notion that autophagy was a non-selective pathway, in which cargos were randomly degraded, growing evidence suggests that autophagy is a highly selective and tightly regulated process that requires cargo recognition and processing by the autophagic machinery. Autophagy selectivity relies on a complex combination between ubiquitin signals, autophagy receptors (such as p62, NBR1, OPTN or NDP52), which are able to recognize and tether specific substrates to phagophores (which will mature into autophagosomes), and AuTophaGy related proteins 8 ("proteins of the ATG8 family" or "ATG8-like proteins" or "ATG8s"), which are anchored in the phagophore membrane. The LC3-interacting region (LIR) motif (also known as LC3 recognition sequence (LRS) or ATG8-interacting motif (AIM)), which is present in autophagy receptors, allows them to interact with protein(s) of the ATG8 family.

Proteins of the ATG8 family are central components of autophagy in most organisms, because of their role in autophagosome formation and maturation, cargo recognition, and the recruitment of cargos into the autophagosomal membrane. In mammals, there are two subfamilies of ATG8-like proteins : microtubule-associated protein light chain 3 (LC3s) proteins (which comprise LC3A, LC3B and LC3C) and y-amino butyric acid receptor-associated (GABARAPs) proteins (which comprise GABARAP, GABARAPL1 and GABARAPL2). Yeast has only one ATG8 homolog, *Caenorhabditis elegans* and *Drosophila melanogaster* have two, whereas *Arabidopsis thaliana* has nine ATG8 homologs (as reviewed in Shpilka et al, Genome Biol. (2011), 12(7): 226).

The mechanisms involving proteins of the ATG8 family are complex and still not fully understood. In particular, little is known about the distinct roles of the different proteins of the ATG8 family, in driving selective autophagy pathway.

Some tools, such as anti-ATG8s antibodies, fluorescently-labelled ATG8s or more recently, tandem probes such as RFP-GFP-ATG8 or GFP-ATG8-RFP-ATG8DG, have been developed to analyze autophagy and/or monitor its dynamics. However, none of these tools can ensure the absence of cellular artifacts due to their overexpression.

Furthermore, the endogenous expression of distinct AGT8 molecules is ignored and might change in each cell type or under distinct stress conditions. Recently, AIM/LIR-based fluorescent sensors, interacting with proteins of the ATG8 family, have been developed to overcome some of the limits of the current tools (Lee et al, EMBO J. (2017), 36(8):1100-1116 ; Stolz et al, EMBO J. (2017), 36(4):549-564). Despite these recent developments, the need remains for tools that allow capture, isolation, purification, detection and identification of endogenous ATG8s proteins in any cellular contexts, as well as to assess their expression levels and cellular localization, and changes which may occur in certain physiological or pathogenic conditions.

It has now been discovered that engineered (poly)peptides comprising a sequence consisting of LIR core motif(s) and specific linker peptidic sequences, which can be used as spacers between LIR core motifs, bind selectively and with high affinity, in the low nanomolar range (particularly when they contain multiple LIRs), to proteins of the ATG8 family. These molecular tools can capture or "trap" ATG8s at endogenous levels, and can for instance advantageously be used to isolate/purify proteins of the ATG8 family, and identify them (and potentially also their interactor(s)), by methods such as Western blot or mass spectrometry. When they are coupled to a detection tag, these small-sized (poly)peptides can also be used to monitor autophagy in an endogenous way in various biological models (such as mammalian cells and *C. elegans*), e.g. as fluorescent probes/sensors to localize endogenous proteins of the ATG8 family within the cells and monitor autophagy by microscopy, without the need for any artificial expression of ATG8s which could affect autophagic flux or disrupt the natural balance between ATG8 molecules observed in various biologic models. The (poly)peptides according to the invention can also be used to identify autophagy inhibitors, in particular using high-throughput screens of small-molecules collections.

### DESCRIPTION OF THE INVENTION

Therefore, the present invention relates to a peptide or polypeptide comprising, consisting essentially of or consisting of a sequence (« pepLIR ») consisting of a LC3-interacting region (LIR) core motif and two linker peptidic sequences, wherein :
- the LIR core motif has a sequence of SEQ ID NO :1
   SEQ ID NO :1 : [F/W/Y]xx[I/L/V]
   wherein x denotes any amino acid, preferably selected among natural amino acids, [F/W/Y] denotes an amino acid selected in the group consisting of phenylalanine (F), tryptophan (W) and tyrosine (Y), and [I/L/V] denotes an amino acid selected in the group consisting of isoleucine (I), leucine (L) and valine (V),
- the first linker peptidic sequence ("N-linker") is linked to the N-ter of the LIR core motif, and consists of six amino acids, wherein
   ∘ at least one amino acid between positions 1 and 5 of the N-linker is an aspartic acid (D), and
   ∘ the amino acid at position 6 of the N-linker is an aspartic acid (D), and
- the second linker peptidic sequence ("C-linker") is linked to the C-ter of the LIR core motif, and consists of two amino acids.

In the context of the present invention:
- A (poly)peptide refers to a polymer of amino acid residues, which are linked together by amide bonds. Amino acids are generally L-amino acids, unless stated otherwise. Amino acids can be naturally occurring or non-naturally occurring, but they are preferably naturally occurring. Here, it is referred to a peptide for a polymer of fewer than 100 amino acids, and to a polypeptide for a polymer of at least 100 amino acids. The terms "peptide" and "polypeptide" also include the possibility of typical post-translational modifications, such as phosphorylation of amino acid residues (e.g. tyrosine (Y)), especially when the (poly)peptides are expressed in eukaryotic cells ;
- The term "consisting of" (and variations such as "consists" and consist"), in the context of a specific amino acid sequence or nucleic acid sequence disclosed herein, is to be understood as meaning including, and limited to, said amino acid sequence or said nucleic acid sequence. The term "comprising" (and variations thereof) is meant to imply the inclusion of at least said amino acid sequence or nucleic acid sequence. The term "consisting essentially of" (and variations thereof) includes within its scope (i) for amino acid sequences, about 1 to about 10 optional amino acids (and all integer optional amino acids in between) upstream of said amino acid sequence and/or about 1 to about 10 optional amino acids (and all integer optional amino acids in between) downstream of said amino acid sequence, and for (ii) nucleic acid sequences, about 3, 6, 9, 12, 15, 18, 21, 24, 27, 30 nucleotides upstream of said nucleic acid sequence and/or about 3, 6, 9, 12, 15, 18, 21, 24, 27, 30 nucleotides downstream of said nucleic acid sequence;
- The percentage of sequence identity between a given sequence (test sequence) and another sequence (reference sequence) is determined by methods well-known to a skilled person. First, the two sequences are first optimally aligned. The two sequences can be of the same size, or of different sizes. In some cases, it may be necessary to add "gaps" in one of the sequence, in order to allow an optimal alignment with the other sequence. The optimal alignment of the two sequences can for instance be carried out using the Smith and Waterman algorithm (Smith and Waterman (1981), J. Theor. Biol., 91 (2): 370-380), the Needleman and Wunsch algorithm (Needleman and Wunsch (1972), J. Mol. Biol, 48(3): 443-453) or the Pearson and Lipman method (Pearson and Lipman (1988), Proc. Natl. Acad. Sri. U. S.A., 85(5): 2444-2448). Some softwares can be useful to implement some of these algorithms, such as GAP, BESTFIT, FASTA, TFASTA (Wisconsin Genetics Software Package Release 7.0, Genetic Computer Group, 575, Science Drive, Madison, Wisconsin), BLAST or CLUSTALW (Thompson et al (1994), Nucleic Acids Res 22(22):4673-80). The best alignment (i.e. the one which allows to get the highest percentage of sequence identity in the comparison window), among the various alignments provided by these various methods, is selected. A comparison is then carried out between respective amino acids located at the same position in the test sequence and in the reference sequence. When a given position is occupied by the same amino acid in the two sequences, these sequences are identical for this position. The percentage of sequence identity is then determined from the number of positions for which respective amino acids are identical, over the total number of amino acids in the reference sequence.
- A conservative substitution corresponds to the replacement of an amino acid by another amino acid having similar physicochemical properties (such as size, charge and/or hydrophobicity). This similarity can be assessed in terms of physicochemical distance between two amino acids, using for instance the Grantham's distance (Grantham (1974), Science, 185 (4154): 862-864), the Sneath's index (Sneath (1966), Journal of Theoretical Biology, 12 (2): 157-195), the Epstein's coefficient of difference (Epstein (1967), Nature. 215 (5099): 355-359), the Miyata's distance (Miyata et al (1979), Journal of Molecular Evolution, 12 (3): 219-236) or the Experimental Exchangeability (Yampolsky and Stoltzfus (2005), Genetics. 170 (4): 1459-1472).

The LIR core motif of four amino acids, as well as amino acids close to the LIR core motif in the linker peptidic sequences, are mostly responsible for the binding/interaction of the (poly)peptides of the present invention with protein(s) of the ATG8 family. The linker peptidic sequences (in particular, the N-linker) allow a better solubility of the (poly)peptides, as well as an optimal spatial conformation and presentation of the LIR core motif(s).

In the following (in particular in the Figures and Examples sections), the (poly)peptides according to the invention may indistinctly be referred to as "traps", "ATG8 traps", "LC3 traps" or "LIR traps".

Preferably, amino acids at position 2 and/or 3 of the sequence of the LIR core motif, as described above, are selected in the group consisting of hydrophobic amino acids (in particular: V, I, L, M), acidic amino acids (D, E) and phosphorylable amino acids (S, T). In a preferred embodiment, the LIR core motif has a sequence selected in the group consisting of FVII (SEQ ID NO: 2), WEEL (SEQ ID NO: 3) and YDVI (SEQ ID NO: 4).

In one aspect of the invention, the peptide or polypeptide comprises, consists essentially of, or consists of a pepLIR sequence, which has at least 90, 91, 92, 93, 94, 95, 96, 97, 98, 99, 100% identity with any one of the following sequences :
SEQ ID NO : 5 : SCDTDDFVIIPH ("pepLIR-F")
SEQ ID NO : 7 : ESLDDDWEELDP ("pepLIR-W")
SEQ ID NO : 9 : DAASRDYDVILE ("pepLIR-Y")

Preferably, the pepLIR sequence as described above is selected in the group consisting of SEQ ID NO : 5, SEQ ID NO : 7 and SEQ ID NO : 9, and sequences wherein one amino acid (at any position of SEQ ID NO : 5, 7 or 9, except positions 6, 7 and 10) is substituted by another amino acid (this substitution can be conservative or non-conservative), and wherein the ability to bind to protein(s) of the ATG8 family is preserved. SEQ ID NO : 6, 8 and 10 are examples of nucleic acid sequences encoding (poly)peptides of SEQ ID NO : 5, 7 and 9, respectively.

Particularly preferred N-linker sequences are selected in the group consisting of :
SEQ ID NO : 11 : SCDTDD ("N-linker F")
SEQ ID NO : 12 : ESLDDD ("N-linker W")
SEQ ID NO : 13 : DAASRD ("N-linker Y")

More preferably, the N-linker of SEQ ID NO: 11, 12, 13, respectively, is linked to the N-ter of the LIR core motif of SEQ ID NO: 5, 7, 9, respectively.

Preferably, the peptide or polypeptide according to the invention, as described above, comprises at least two pepLIR sequences. In particular, the peptide or polypeptide according to the invention can comprise two, three, four, five, six, seven, eight, nine, ten, eleven or twelve pepLIR sequences. The higher the number of pepLIR sequences, the higher the affinity of the peptide or polypeptide according to the invention towards protein(s) of the ATG8 family. However, if the number of pepLIR sequences is too high, the solubility and stability of the (poly)peptide may decrease. Moreover, pepLIR sequences displayed in tandem (i.e. pepLIRs in pairs) are more convenient to assess the cooperative effect and facilitate the cloning. It is therefore particularly advantageous to use two, four or eight pepLIR sequences. More preferably, when the peptide or polypeptide according to the invention comprises at least two pepLIR sequences, said pepLIR sequences can be directly linked to each other or linked by a peptidic linker of one, two or three amino acids; this leads to (poly)peptides which have an optimal degree of flexibility, high affinity for proteins of the ATG8 family and good stability (in particular, in terms of solubility and susceptibility to proteolytic attack).

In another aspect of the invention, the peptide or polypeptide comprises, consists essentially of, or consists of a sequence ("pepLIR2"), which has at least 80, 81, 82, 83, 84, 85, 86, 87, 88, 89, 90, 91, 92, 93, 94, 95, 96, 97, 98, 99, 100% identity with any one of the following sequences :
SEQ ID NO : 14 : SCDTDDFVIIPHSCDTDDFVIIPH ("pepLIR2-F")
SEQ ID NO : 16 : ESLDDDWEELDPESLDDDWEELDP ("pepLIR2-W")
SEQ ID NO : 18 : DAASRDYDVILEDAASRDYDVILE ("pepLIR2-Y")

Preferably, the pepLIR2 sequence is selected in the group consisting of SEQ ID NO : 14, SEQ ID NO : 16 and SEQ ID NO : 18, and sequences wherein one, two, three or four amino acid(s) (at any position of SEQ ID NO : 14, 16 or 18, except positions 6, 7, 10, 18, 19 and 22) is(are) substituted by another amino acid (this(these) substitution(s) can be conservative or non-conservative) and/or wherein one, two or three amino acid(s) is(are) inserted between positions 12 and 13 of SEQ ID NO : 14, 16 or 18, and wherein the ability to bind to protein(s) of the ATG8 family is preserved. Advantageously, GS sequences may be added in N-ter and/or in C-ter of the pepLIR2 sequence. SEQ ID NO : 15, 17 and 19 are examples of nucleic acid sequences encoding (poly)peptides of SEQ ID NO : 14, 16 and 18, respectively.

Preferably, the peptide or polypeptide comprising, consisting essentially of, or consisting of a pepLIR2 sequence, as described above, comprises, consists essentially of, or consists of one, two, three, four, five or six pepLIR2 sequences, more preferably one, two or four pepLIR2 sequences.

When the peptide or polypeptide according to the invention comprises, consists essentially of or consists of at least two pepLIR2 sequences, said pepLIR2 sequences are preferably directly linked to each other or are linked by a peptidic linker of one, two or three amino acids.

The present invention also relates to a peptide or polypeptide as described above, in any embodiment, which further comprises a tag, said tag being preferably a detection and/or purification tag. Said tags are well-known to the one skilled in the art. As examples of tags, we can cite polyhistidine tags (His-tags) (for example H6 and H10), Glutathione S-Transferase (GST) fusions, Maltose Binding Protein (MBP) fusions, SV5 epitope tags, c-myc tags, FLAG-tags, streptavidin-tags, as well as biotin or fluorescent molecules. These tags can be expressed directly with the peptide or polypeptide according to the invention, when the tag is of peptidic nature, or conjugated to the peptide or polypeptide according to the invention, using methods which are well-known to the one skilled in the art. Examples of (poly)peptides with tags according to the invention are presented in the examples (see Table 1).

Further objects of the invention include :
- a nucleotide sequence encoding a peptide or polypeptide as described above, in any embodiment. Examples of such nucleotide sequences are disclosed in SEQ ID NO: 6, 8, 10, 15, 17, 19, as well as in the examples (see Table 1) ;
- a gene construct comprising such a nucleotide sequence. In a particular embodiment, said gene construct is operatively bound to transcription, and optionally translation, control elements. The gene construct of the invention can incorporate an operatively bound regulatory sequence of the expression of the nucleotide sequence of the invention, thus forming an expression cassette. As used in this description, the expression "operatively bound" means that the polypeptide of the invention, encoded by the nucleotide sequence of the invention, is expressed in the correct reading frame under the control of expression control, or regulatory sequences. Control sequences are sequences controlling and regulating the transcription and where appropriate, the translation of the (poly)peptide of the invention, and include promoter sequences, transcriptional regulator-encoding sequences, ribosome-binding sequences (RBS) and/or transcription termination sequences. In a particular embodiment, said expression control sequence is functional in prokaryotic organisms and cells, for example, bacteria, etc., whereas in another particular embodiment, said expression control sequence is functional in eukaryotic organisms and cells, for example, insect cells, plant cells, mammal cells, etc. Examples of well-known promoters suitable for carrying out the invention include constitutive promoters such as those found in some eukaryotic viruses (polyoma virus, adenovirus, SV40, CMV, avian sarcoma virus, hepatitis B virus, metalothionein gene promoter, herpes simplex virus thymidine kinase promoter, retroviral LTR regions, immunoglobulin promoter, actin promoter, EF-lalpha promoter), as well as inducible promoters wherein expression of the downstream gene requires addition of a substance of an exogenous signal to the culture such as the tetracycline promoter, NFKappaB/UV light, Cre/lox, heat shock promoters, regulable RNA polymerase II promoters described in WOP2006/135436, as well as tissue-specific promoters, such as the PSA promoter described in WO2006012221. Preferably, inducible promoters are used. Advantageously, the construct of the invention further comprises a marker or gene encoding a motif or a phenotype, which allows screening the host cell transformed with said construct. In a particular embodiment, the expression of said gene construct is externally controlled. In a more particular embodiment, the expression is externally controlled using the doxycycline Tet-On system ;
- a vector comprising such a nucleotide sequence or gene construct. The choice of the vector will depend on the host cell in which it is to be subsequently introduced. In a particular embodiment, the vector of the invention is an expression vector. Suitable vectors for the insertion of the nucleotide sequence of the invention or the gene construct of the invention are vectors derived from prokaryotic expression vectors such as pUCl8, pUCl9, pGEX-6P1, Bluescript and their derivatives, mpl8, mpl9, pBR322, pMB9, ColEl, PCRI, RP4, phage and "shuttle" vectors such as pSA3 and pAT28, yeast expression vectors such as yeast 2 micron plasmid, integration plasmid, yeast episomal plasmid O(Ep) vectors, similar and centromeric plasmids, expression vectors in insect cells such as vectors the pAC series and the pVL series, plant expression vectors such as vectors series pIBI, pEarleyGate, pAVA, pCAMBIA, pGSA, pGWB, pMDC, pMY, pORE and similar and expression vectors in eukaryotic cells rather than based on viral vectors (adenovirus, adenovirus associated viruses and retroviruses, and particularly, lentivirus) and non-viral vectors such as pSilencer 4.1-CMV (Ambion), pcDNA3, pcDNA3.l/hyg pHCMV/Zeo, pCR3.l, pEFI/His, pIND/GS, pRc/HCMV2, pSV40/Zeo2, PTRACE-HCMV, pUB6N5-His, pVAXl, pZeoSV2, pCI, pSVL and pKSV-10, pBPV-I, pML2d and pTDTI. By way of illustration, the vector in which said nucleic acid sequence is introduced can be a plasmid which is or is not integrated in the genome of a host cell when it is introduced in said cell. Illustrative, non-limiting examples of vectors in which the nucleotide sequence of the invention or the gene construct of the invention can be inserted include a Tet-On inducible vector for expression in eukaryotic cells ;
- a cell comprising such a nucleotide sequence, gene construct or vector. The host cell can be prokaryotic or eukaryotic (e.g. bacteria, mammalian cells, yeast cells, fly cells, plant cells...) ;
- a non-human animal comprising such a nucleotide sequence, gene construct, vector or cell. Examples of such animals include *C. elegans,* zebrafish, drosophila and mammals (preferably a rodent, more preferably a mouse or a rat).

The present invention further relates to a process for obtaining a peptide or polypeptide as described previously, in any embodiment, comprising :
- a step of culturing a cell comprising a nucleotide sequence, gene construct or vector, as described above, under conditions which allow producing said peptide or polypeptide. Conditions for optimizing the culture of said cell will depend on the cell used and are well known to the skilled person in the art, or
- a step of peptide synthesis, preferably using solid-phase peptide synthesis methods, which are well-known to the one skilled in the art.

Preferably, the peptide or polypeptide of the present invention is prepared using recombinant DNA techniques (which allow cheap production, in large quantities, of (poly)peptides), for example by a procedure including the steps of :
(a) preparing a vector (typically, an expression vector) comprising a nucleotide sequence that encodes the peptide or polypeptide of the invention and that is operably linked to a regulatory element, such as transcriptional and/or translational regulatory nucleic acids ;
(b) introducing the vector into a host cell (e.g. a bacteria);
(c) culturing the host cell to express the nucleotide sequence to thereby produce the encoded peptide or polypeptide of the invention; and
(d) optionally, recovering/isolating the peptide or polypeptide of the invention. Standard protocols for recombinant methods are for example described in Klint, et al. (2013) PLOS One, 8(5): e63865; Sambrook, et al. (1989) Molecular Cloning: A Laboratory Manual (Cold Spring Harbour Press), in particular Sections 16 and 17; Ausubel, et al. (1998) Current Protocols in Molecular Biology (John Wiley and Sons, Inc.), in particular Chapters 10 and 16; and Coligan, et al. (1997) Current Protocols in Protein Science (John Wiley and Sons, Inc.), in particular Chapters 1, 5 and 6.

The present invention also relates to a kit, for instance a diagnostic kit, comprising a peptide or polypeptide of the present invention, as described above, in any embodiment, a nucleotide sequence as described above, in any embodiment, a gene construct as described above, in any embodiment, or a vector as described above, in any embodiment.

The present invention also relates to the use of a peptide or polypeptide as described above, of a nucleotide sequence as described above, of a gene construct as described above, of a vector as described above, or of a kit as described above :
- for the capture/isolation/purification of protein(s) of the ATG8 family and optionally of their interactor(s) (e.g. in GST pulldown experiments). Proteins of the ATG8 family and/or their interactor(s) can then optionally be identified by methods well-known to the one skilled in the art, such as Western blot or mass spectrometry. For this use of capture, (poly)peptides according to the present invention generally comprise a purification tag (for instance, a GST tag, which may allow purification on a glutathione-sepharose column or glutathione-agarose beads) ;
- for the detection of protein(s) of the ATG8 family. For this use, (poly)peptides according to the present invention generally comprise a detection tag. The (poly)peptides of the invention can for instance be used to monitor autophagy, in any physiologic or pathologic condition, in an endogenous way in various biological models (such as mammalian cells and *C. elegans*), e.g. as fluorescent probes/sensors to localize endogenous proteins of the ATG8 family within the cells and monitor autophagy by microscopy, without affecting the autophagic flux. The (poly)peptides according to the present invention can also be used to quantify proteins of the ATG8 family in cell extracts or biological samples, e.g. using immunoassays, such as ELISA, DELFIA^{®}, AlphaLISA, EIA, RIA, Western Blot, Lateral Flow Assays, immunohistochemistry, Protein Arrays or Immuno-PCR ; or
- for the screening/identification (in particular using high-throughput screens) of molecules which inhibit specific autophagy, by acting on the interaction between a protein of the ATG8 family and the peptide or polypeptide of the invention, competing with endogenous proteins involved in the targeted process. For this use of screening, the peptide or polypeptide of the invention can be used for the capture or the detection.

### BRIEF DESCRIPTION OF THE DRAWINGS

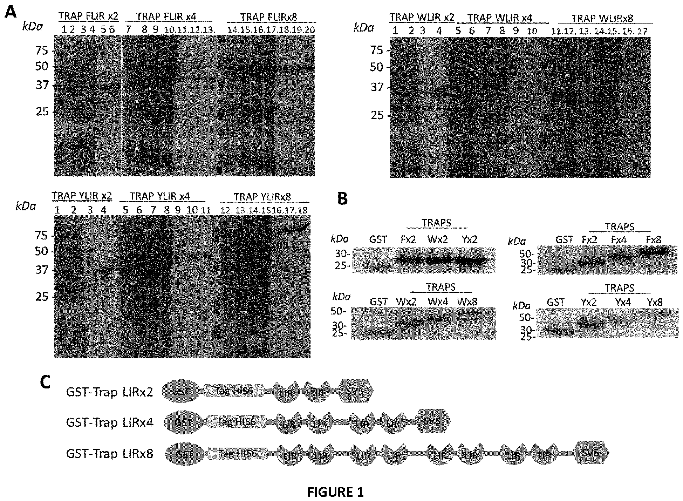

**Figure 1****. LC3s traps containing LIR disposed in tandem and their purification.** The traps Fx2, Yx2, Wx2 are monomeric traps (1 insert, two LIRs) carrying the consensus F, W and Y respectively. Traps containing two inserts (4 LIRs) or four inserts (8 LIRs) were cloned and named Fx4, Wx4, Yx4 or Fx8, Wx8, Yx8 depending on the number of inserts. **A)** Coomassie of the fractions collected from proteins purified from bacteria. The first 3-4 fractions contain total extracts or unbound proteins. The next 2-3 fractions contain purified proteins after dialysis. **B)** Western blot against SV5 tag analysis of the distinct LC3 traps. The antibody SV5 recognizes the C-terminus of these proteins according to the illustration. C) Representation of the structures of the traps. The traps F, W and Y carry the LIR sequences.

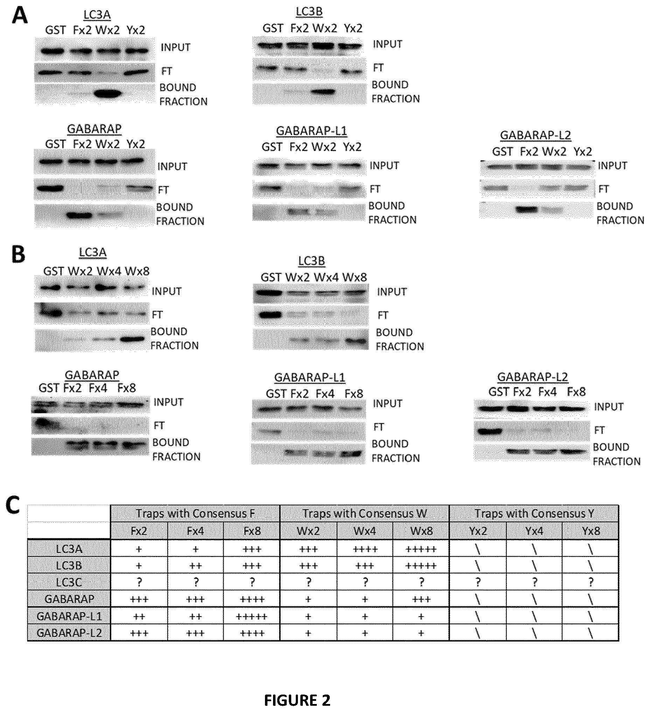

**Figure 2****. Isolation of ATG8s proteins from mammalian cell extracts using the LC3 traps.** Extracts from 20 x 10⁶ of ZBR cells treated with Bafilomycin A were incubated with distinct GST-LC3 traps and GST pulldown experiments were performed. ATG8s proteins were analyzed by Western blot with different specific antibodies. Input, flow through (FT) and bound fractions were analyzed. **A)** LC3 traps F, W and Y containing 1 insert (2 LIRs) were compared in their capacity to pull down LC3/GABARAP family members. LC3C was not analyzed because in these cells this protein shows low level of expression. **B)** LC3-traps containing 1, 2 and 4 inserts (2, 4 and 8 LIRs respectively) were used to explore the cooperative effect of LIRs after multiplication to capture LC3/GABARAP family members. **C)** Qualitative results obtained from the capture of LC3/GABARAP members using distinct LC3-traps. Interactions are qualitatively recorded in the table as (+) low to (+++++) high interactions. No interaction is indicated as (\).

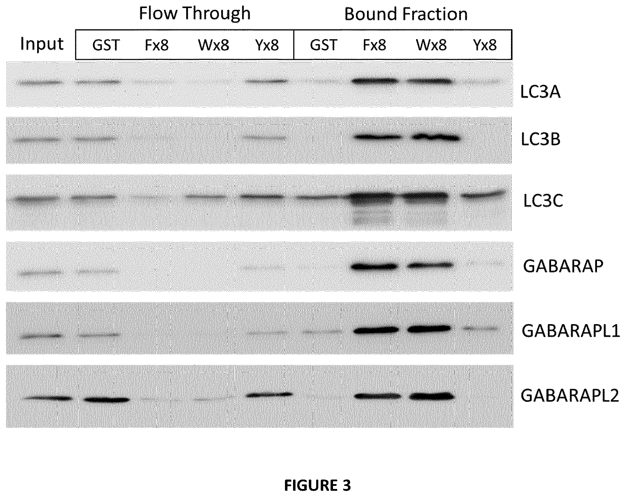

**Figure 3****. *In vitro* isolation of recombinant ATG8s proteins using LC3 traps.** GST pulldown assays were used to analyze the capacity of distinct LC3-traps to capture each of the LC3/GABARAP proteins. Recombinant LC3-traps (5 µg) were used to capture 1 µg of each LC3/GABARAP proteins. One-hour binding time was used. Input, Flow Through and Bound Fractions were analyzed by Western blot with the indicated specific antibodies.

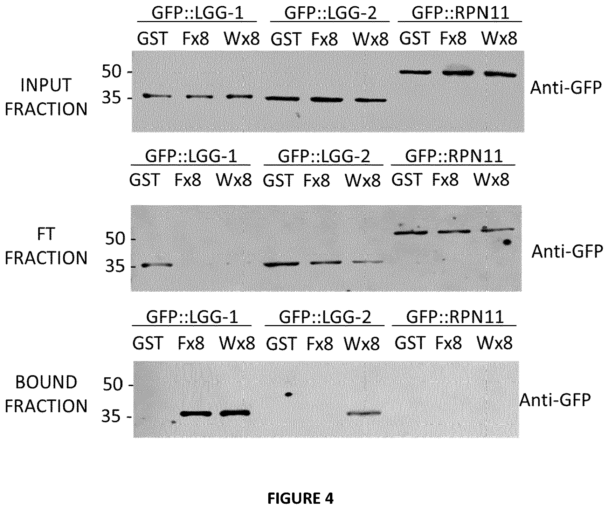

**Figure 4****. *In vivo* capture of LGG-1 and LGG-2 from** ***C.** Elegans* **using LC3 traps.** LGG-1 (GABARAP homolog) and LGG-2 (LC3 homolog) were purified using tetrameric F and W LC3 traps from 3 different *C. Elegans* strains as indicated (500 µg of total proteins from lysates were used per sample). GST pulldown was performed as previously indicated and collected fractions were analyzed by Western blot using an anti-GFP.

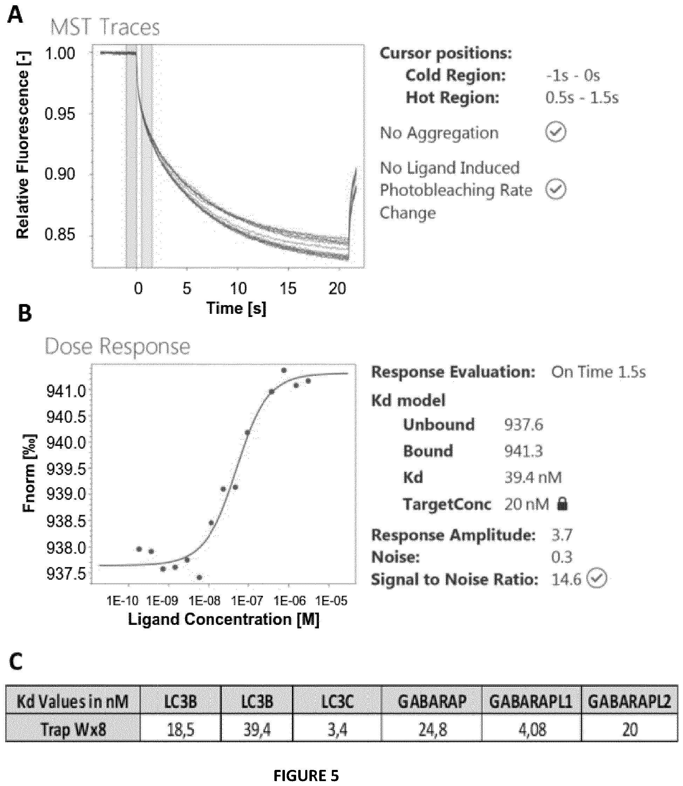

**Figure 5****. MST results: affinities measurements of Wx8 traps with ATG8s proteins.** Thermophoresis was used to find K_{d} to evaluate the affinities of our traps toward each members of ATG8 proteins family. **A)** Relative Fluorescence against the time of the 16 binding reactions. These binding reactions mixed the traps Wx8 at constant concentration 20nM and LC3B diluted from 3µM to 91nM. **B)** Normalized fluorescence against the ligand concentration, here LC3B. From this curve response, the K_{d} is extracted to give information on the interaction between the two partners. **C)** Table of the K_{d} found between Trap Wx8 and the recombinant ATG8s.

### EXAMPLES

The present invention is illustrated non-exhaustively by the following examples. These examples are intended for the purpose of illustration only and are not intended to limit the scope of the present invention.

### Example 1: Cloning, expression and purification of LC3 traps

Three types of traps were constructed :
- LIR-traps F (comprising one, two or four inserts, comprising respectively two, four, or eight pepLIR-F sequences of SEQ ID NO : 5 : SCDTDDFVIIPH, with a LIR core motif of SEQ ID NO: 2 : FVII),
- LIR-traps W (comprising one, two or four inserts, comprising respectively two, four, or eight pepLIR-W sequences of SEQ ID NO : 7 : ESLDDDWEELDP , with a LIR core motif of SEQ ID NO: 3 : WEEL), and
- LIR-traps Y (comprising one, two or four inserts, comprising respectively two, four, or eight pepLIR-Y sequences of SEQ ID NO : 9 : DAASRDYDVILE, with a LIR core motif of SEQ ID NO: 4 : YDVI).

### Material and Methods

### - Cloning

Single-strand DNA were generated by Eurogentec. Oligos were designed to expose in 5' BglII and BamHI in 3' for a direct cloning into the BamHI site of the pGEX-6P1 vector (GE Healthcare Life Sciences) modified with an SV5 tag as reported previously (Hjerpe R et al EMBO Rep. (2009), 10(11):1250-8). These LIR coding sequences were ligated with themselves to generate multiple insert sizes. The previously digested and purified vector was directly added to the ligation mix containing the pool of LIR sequences. These two ligation steps were performed separately for each of the three LIR sequences. After DNA precipitation/concentration, the three ligation reactions were transformed in electrocompetent DH5α cells. Fourty positives clones for each of the three LC3 traps were selected for a total of 120 screened clones. As the plasmid contained the antibiotic (Ampicillin) resistance, colonies were selected and maintained in LB media with this antibiotic. The PCR screening gave information on the number of LIR inserts cloned into the pGEX-6P1-SV5 plasmid, according to the size of the amplified fragment. PCR positive clones containing one, two or four inserts were transformed in chemocompetent BL-21 cells, for protein expression by induction with IPTG. Cell lysate from those cultures were analysed by Western blotting with anti-SV5. The positive clones expressing the SV5 tag were sent for DNA sequencing. For each of the three LIR motifs, three traps with 1, 2 and 4 LIR cloned inserts were selected for a larger protein production, as described below.

### - Protein expression and purification

The LC3 traps were expressed in *E. coli* BL-21 strain and purified by affinity chromatography using a glutathione agarose matrix as previously reported (Hjerpe et al, EMBO Rep. (2009),10(11):1250-8; Aillet et al, Methods Mol Biol. (2012), 832:173-83). All proteins were expressed in *E. coli* BL-21 with IPTG induction (1mM) for 16h at 20°C. Cells were lyzed by sonication in Lysis buffer (PBS, 0.5M NaCL, 2mM benzamidine, 1mM PMSF). Lysates were sonicated on ice (6 times 30 seconds pulses) and supplemented with 1% Triton. After 2h centrifugation at 20,000 r.p.m., supernatant was subjected to standard glutathione agarose affinity purification (Sigma-Aldrich, St Louis, MO, USA) according to the manufacturer's instructions. Samples were dialyzed using 12kDa tubing (Sigma-Aldrich, St Louis, MO, USA) and concentrated at 2µg/µl using Amicon Ultra with 3 KDa MWCO (Millipore). Purified LC3 traps were quantified by Nanodrop (280 nm absorbance) and supplemented with 10% glycerol before storing at -80°C.

### Results

For each type of LC3 traps, inserts were cloned into a GST vector, as indicated above. After cloning, these inserts were multiplied to generate 2 or 4 inserts. The final constructs contained 2, 4 or 8 pepLIR sequences for each type of LC3-trap (Figure 1C). Nucleic acid sequences and amino acid sequences corresponding to the various LC3 traps, including GST tag, His6 tag and SV5 tag, are indicated in table 1.

**Table 1. Nucleic acid and amino acid sequences of the various LIR traps (F, W and Y), containing two (x2), four (x4) ou eight (x8) pepLIRs, and GST, His6 and SV5 tags.**

| | **Amino acid sequence** | **Nucleic acid sequence** |
|---|---|---|
| LIR-trap Fx2 | SEQ ID NO: 20 | SEQ ID NO: 21 |
| LIR-trap Fx4 | SEQ ID NO: 22 | SEQ ID NO: 23 |
| LIR-trap Fx8 | SEQ ID NO: 24 | SEQ ID NO: 25 |
| LIR-trap Wx2 | SEQ ID NO: 26 | SEQ ID NO: 27 |
| LIR-trap Wx4 | SEQ ID NO: 28 | SEQ ID NO: 29 |
| LIR-trap Wx8 | SEQ ID NO: 30 | SEQ ID NO: 31 |
| LIR-trap Yx2 | SEQ ID NO: 32 | SEQ ID NO: 33 |
| LIR-trap Yx4 | SEQ ID NO: 34 | SEQ ID NO: 35 |
| LIR-trap Yx8 | SEQ ID NO: 36 | SEQ ID NO: 37 |

LC3 traps were purified using standard GST protein purification procedures. The final fraction contained a dominant single band with a size of 35kDa for a monomer (containing 2 pepLIRs), 40kDa for a dimer (containing 4 pepLIRs) or 50kD for a tetramer (containing 8 pepLIRs) (Figure 1A). These proteins could be detected with an anti GST or SV5 antibody as illustrated in Figure 1B.

### Example 2: LC3 traps can capture proteins of the ATG8 family (LC3/GABARAP) from cell extracts of lymphoma cells

To investigate the capacity of LC3 traps to capture distinct LC3/GABARAP family members in mammalian cells, GST pulldown experiments were performed.

### Material and Methods

### - Material and reagents

Antibodies for LC3A (4599), LC3B (2775), LC3C (14736), GABARAP (11876), GABARAP L1 (14256) GABARAPL2 (14256) antibodies were purchased from Cell Signaling Technology.

### - Cell culture

Mantle Cell Lymphoma ZBR cells were cultured in RPMI 1640 with 2 mM L-glutamine, 100 Units/mL penicillin, 100 µg/mL streptomycin and 10% fetal bovine serum (FCS). Cells were incubated at 37°C, 5% CO₂. Cell lines authentication was based on short tandem repeat (STR) profiling by DSMZ services (Braunschweig, Germany).

### - Pulldown/capture

Pulldown experiments were performed essentially as described previously (Hjerpe et al, EMBO Rep. (2009),10(11):1250-8; Aillet et al, Methods Mol Biol. (2012), 832:173-83), with minor modifications as indicated in the following. Briefly, for these *in vivo* pulldown experiments, 20 x 10⁶ ZBR cells treated 8h with 20nM BafilomycinA (in order to block autophagy and accumulate ATG8s proteins) were incubated on ice 10 minutes in lysis buffer [50 mM sodium fluoride, 5 mM tetra-sodium pyrophosphate, 10 mM β-glyceropyrophosphate, 1% Igepal CA-630, 2 mM EDTA, 20 mM Na₂HPO₄, and 1.2 mg/mL complete protease inhibitor cocktail (Roche, Basel, Switzerland)] supplemented with 100µg of the different LC3 traps or GST, used as a control. After cold centrifugation at 16,200 g for 30 min, the supernatant was recovered. Lysates were added to 200µL of prewashed/binding buffer equilibrated glutathione-agarose beads (Biontex, Martinsried, Germany), either for 6h or overnight at 4°C. Beads were centrifugated at 1000 g for 5 min (Beckman Coulter Microfuge 22R, Fullerton, CA, USA), and 1/10 of the unbound fraction or flow-through (FT) was saved for Western blot analysis. Three to four washes were carried out using 10 column volumes of wash buffer (PBS-tween 0.05%). Sample elution was done in 100 microliters of Laemmli Buffer 3X. For Western blot analysis, input, flow through and eluted fractions were migrated in 15% acrylamide gels, transferred into PVDF membranes (Immobilon), and blotted against the indicated antibodies (referred in Material and reagents). Membranes were revealed by chemiluminescence (LICOR Odyssey).

### Results

The presence of all ATG8s proteins was analyzed by Western blot in the different fractions as indicated in Figure 2. Our results indicate that LC3-traps containing a single insert (2 LIRs) have different preferences for distinct LC3/GABARAP family members (Figure 2A). While LC3-trap W prefers to interact with LC3 A and B proteins, LC3-trap F prefers to interact with GABARAPs proteins from cell extracts. LC3-traps Y do not seem to interact with any of these proteins. The levels of the LC3C protein in ZBR cells was too low, so that we could not assess its capacity to interact with any of our traps in these pull-down experiments. The multiplication of domains to 2 or 4 inserts (4 and 8 LIRs respectively) results in a cooperative effect in the capacity of LC3-trap W to capture LC3 proteins A and B (Figure 2B). The cooperative effect of multiple LIRs of the LC3-trap F was less obvious to observe with GABARAP and GABARAPL2 but it was well observed with GABARAPL1. These results are recapitulated in Figure 2C.

### Example 3: LC3 traps can capture recombinant proteins of the ATG8 family (LC3/GABARAP)

The interactions observed using cell extracts (as in example 2) could have been indirect interactions or regulated by cofactors present in cell extracts. To investigate the capacity of LC3 traps to directly interact with distinct LC3/GABARAP (ATG8s) family members, GST pull down experiments were performed using recombinant LC3/GABARAP (ATG8s) proteins purified from bacteria.

### Material and Methods

### - Material and reagents

Recombinant Human Nterm His6 tagged LC3A (UL-430), Nterm His6 tagged Apg8p1/GABARAP (UL-410), Nterm His6 tagged Apg8p2/GABARAPL2 (UL-420) were purchased from BostonBiochem. Recombinant Human Nterm His6 tagged LC3B (NBP1-99115) and recombinant human Nterm His6 tagged GABARAPL1 (NBP1-50916) were purchased from Novusbio. Recombinant Human Nterm His6 tagged MAP1LC3C (CSB-EP887153HU) were purchased from Cusabio.

Antibodies for LC3A (4599), LC3B (2775), LC3C (14736), GABARAP (11876), GABARAP L1 (14256) GABARAPL2 (14256) antibodies were purchased from Cell Signaling Technology.

For these *in vitro* capture/pulldown experiments, 5 µg of each trap (containing four inserts (8 LIRs)) were used to pull down 1µg of recombinant ATG8s proteins, in PBS star binding buffer [PBS 1X, 0.1% Igepal, 2mM DTT]. Traps and free ATG8s recombinant proteins were incubated 1h rotating at 4°C with Gluthatione-S transferase beads (Sigma). Four washes of 1 mL each were performed with 4°C PBS 0.05% Tween 20 (Sigma), after which bound proteins were eluted by incubation with 100 microliters of Laemmli Buffer 3X. For Western blot analysis, input, flow through and eluted fractions were migrated in 15% acrylamide gels, transferred into PVDF membranes (Immobilon), and blotted against the indicated antibodies (referred in Material and reagents). Membranes were revealed by chemiluminescence (LICOR Odyssey).

### Results

In contrast to the previous experiment using cell extracts (Example 2), LC3/GABARAP proteins produced in bacteria are not conjugated to lipids (lipidated), because the machinery required to do so does not exist in bacteria. In consequence, proteins of the ATG8 family cannot be integrated within autophagosomes, which might affect the interaction capacities of distinct LC3-traps.

The results obtained in these *in vitro* pulldown experiments indicate that LC3-traps containing four inserts (8 LIRs) that showed preferences for LC3 (LC3-Trap W) or GABARAP (LC3-Trap F) *in vivo* did not show much preferences for distinct recombinant LC3/GABARAP family members *in vitro* (Figure 3). This could be due to the absence of molecular interferences/regulatory mechanisms not reconstituted *in vitro.* Interestingly, the trap with Y consensus seemed to interact with recombinant LC3C, and to a lesser extent with GABARAP and GABARAPL1.

### Example 4: LC3 traps can capture proteins of the ATG8 family from another biologic model (Caenorhabditis elegans)

To investigate whether our LC3 traps could be used to study ATG8-regulated processes from other organisms, *in vivo* experiments were performed with *C. elegans. C. elegans* contains only two members of the ATG8 family : LGG-1 (GABARAP homolog) and LGG-2 (LC3 homolog).

### Material and Methods

Three different *C. elegans* strains were generated from the N2 strain and used : one strain stably expressing GFP::LGG-1 (DA2123 strain, as described in Meléndez et al (2003), Science 301(5638):1387-91 and Kang et al (2007), Genes Dev. 21(17):2161-71) and a second strain expressing GFP::LGG-2 (VIG9 strain, as described in Alberti et al (2010), Autophagy 6(5):622-33 and Al Rawi et al (2011), Science 25:334(6059):1144-7) to allow detection of LGG molecules by immunoblotting using an anti-GFP (Santa-cruz sc-9996),as well as a third strain expressing GFP::RPN11, used as a control for nonspecific binding of our traps.

This *in vivo* pulldown experiment was performed as described in Example 2, except that 500µg of total proteins from *C. Elegans* lysate (instead of ZBR cells) were used. The lysis was carried out in the same buffer as described previously, in a Precellys apparatus, using Sigma glass beads 425-600 µm or Precellys ceramic (zirconium oxide) mix beads 1.4 mm and 2.8 mm.

The traps used in this experiment were tetrameric (8 LIRs) F and W LC3 traps.

### Results

Interactions of LC3 traps with LGG-1 and LGG-2 proteins were investigated using pulldown experiments with cell extracts, and results are shown in Figure 4. Our results indicate that LC3-trap W interacts with both LGG-1 and LGG-2, while LC3-trap F interacts only with LGG-1. These results coincide with the results observed with cell extracts from lymphoma cells in Example 2, even if differences observed with LC3-traps F and W to capture LGG1 (GABARAP-like molecules) was not obvious. This could be due to the high levels of expression of LGG1 expressed in the used strain.

### Example 5: Quantification of the affinities and specificities of LC3 traps towards proteins of the ATG8 family

In order to quantify the affinity of our LC3-traps to interact with distinct ATG8s members, we used Micro Scale Thermophoresis (MST), a technique used to quantify the affinity of two interacting partners.

### Material and Methods

Thermophoresis was performed with a Monolith NT.115 machine (Nanotemper). LC3 traps were previously dyed covalently using the Monolith Protein Labeling RED-NHS 2^{nd} Generation kit from Nanotemper. Affinities were measured between the NHS-dyed traps and each of the ATG8s recombinant proteins. To determine each K_{d}, recombinant ATG8s proteins were diluted to prepare 16 samples at different concentrations, and placed with a constant concentration (20 nM) of fluorescent traps, containing four inserts (8 LIRs). Recombinant human Nterm His6 tagged LC3A (UL-430), Nterm His6 tagged Apg8p1/GABARAP (UL-410), Nterm His6 tagged Apg8p2/GABARAPL2 (UL-420) were purchased from BostonBiochem. Recombinant Human Nterm His6 tagged LC3B (NBP1-99115) and recombinant human Nterm His6 tagged GABARAPL1 (NBP1-50916) were purchased from Novusbio. Recombinant Human Nterm His6 tagged MAP1LC3C (CSB-EP887153HU) were purchased from Cusabio.

LC3A was used to determine MST within dilution from 6.5µM to 0.2µM, LC3B from 3µM to 91nM, LC3C from 1µM to 30nM, GABARAP from 60µM to 1.8µM, GABARAP-L1 from 12µM to 0.36µM, GABARAP-L2 from 20µM to 0.6µM. The interaction partners (traps and ATG8s proteins, respectively) were mixed and measured after equilibrium in binding buffer:
- binding buffer used for traps-LC3A and traps-GABARAPL2 interactions : (25mM Hepes pH8.0, 90mM NaCl, 5% Glycerol, 0.5mM DTT, 0.05% Tween, 1.85mM KCI, 5mM Na2HPO4, 0.9mM KH2PO4) ;
- binding buffer used for traps-LC3B and traps-GABARAPL1 interactions : (10mM Tris HCI pH8.0, 115mM NaCl, 5% Glycerol, 0.5mM DTT, 0.05% Tween, 1.85mM KCI, 5mM Na2HPO4, 0.9mM KH2PO4) ;
- binding buffer used for traps-GABARAP interactions : (25mM HEPES pH8.0, 90mM NaCl, 2.5% Glycerol, 0.5mM DTT, 0.05% Tween, 1.85mM KCI, 5mM Na2HPO4, 0.9mM KH2PO4) ;
- binding buffer used for traps-LC3C interactions : (10mM Tris HCI pH8.0, 90mM NaCl, 10% Glycerol, 0.05% Tween, 1.85mM KCI, 5mM Na2HPO4, 0.9mM KH2PO4).

Samples were loaded using Monolith NT.115 Premium Capillaries (Nanotemper), temperature was stabilized at 22°C and excitation laser power set at 60%. Time response to temperature gradients were measured and K_{d} values were fitted to curve responses using the MO.Affinity Analysis software (Nanotemper).

### Results

The time response to temperature gradient of traps-ATG8s complexes were measured and K_{d} were defined (Figure 5). Interestingly, the tetrameric traps (8 LIRs) with the W-LIR consensus showed a same range of affinity toward all ATG8 proteins, around 10 nM. This strong affinity could explain our *in vitro* capture results (Example 3) towards all the ATG8s molecules. However, that is not what we observed *in vivo.* Indeed, W-traps revealed a strong preference for LC3A and LC3B, but low binding to GABARAPs proteins (Example 2). This suggests that other factors (such as interactor, competition with endogenous proteins, lipidation of ATG8s, different protein conformation) found only *in vivo* may have an impact in the capture capacity of our traps toward different ATG8 proteins.

## Claims

1. A peptide or polypeptide comprising a sequence (« pepLIR ») consisting of a LC3-interacting region (LIR) core motif and two linker peptidic sequences, wherein :
- the LIR core motif has a sequence of SEQ ID NO : 1,
- the first linker peptidic sequence (« N-linker ») is linked to the N-ter of the LIR core motif, and consists of six amino acids, wherein
∘ at least one amino acid between positions 1 and 5 of the N-linker is an aspartic acid (D), and
∘ the amino acid at position 6 of the N-linker is an aspartic acid (D), and
- the second linker peptidic sequence (« C-linker ») is linked to the C-ter of the LIR core motif, and consists of two amino acids.

2. A peptide or polypeptide according to claim 1, wherein the pepLIR sequence has at least 90% identity with any one of the sequences SEQ ID NO : 5, 7 and 9.

3. A peptide or polypeptide according to claim 1 or 2, wherein the pepLIR sequence is selected in the group consisting of SEQ ID NO : 5, 7 and 9.

4. A peptide or polypeptide according to any one of claims 1 to 3, which comprises at least two pepLIR sequences.

5. A peptide or polypeptide according to any one of claims 1 to claim 4, which comprises two, four or eight pepLIR sequences.

6. A peptide or polypeptide according to claim 4 or 5, wherein the pepLIR sequences are directly linked to each other or are linked by a peptidic linker of one, two or three amino acids.

7. A peptide or polypeptide according to claim 1, comprising a sequence (« pepLIR2 ») having at least 80% identity with any one of the sequences SEQ ID NO : 14, 16 and 18.

8. A peptide or polypeptide according to claim 7, wherein the pepLIR2 sequence is selected in the group consisting of SEQ ID NO : 14, 16 and 18.

9. A peptide or polypeptide according to claim 7 or 8, which comprises at least two pepLIR2 sequences, and preferably two or four pepLIR2 sequences.

10. A peptide or polypeptide according to claim 9, wherein the pepLIR2 sequences are directly linked to each other or are linked by a peptidic linker of one, two or three amino acids.

11. A peptide or polypeptide according to any of the preceding claims, further comprising a tag, said tag being preferably a detection or purification tag.

12. A nucleotide sequence encoding a peptide or polypeptide according to any of the preceding claims.

13. A gene construct comprising a nucleotide sequence according to claim 12.

14. A vector comprising a nucleotide sequence according to claim 12 or a gene construct according to claim 13.

15. A cell comprising a nucleotide sequence according to claim 12, a gene construct according to claim 13 or a vector according to claim 14.

16. A non-human animal comprising a nucleotide sequence according to claim 12, a gene construct according to claim 13, a vector according to claim 14 or a cell according to claim 15.

17. A process for obtaining a peptide or polypeptide according to any one of claims 1 to 11 comprising :
- a step of culturing a cell according to claim 15 under conditions which allow producing said peptide or polypeptide, or
- a step of peptide synthesis, preferably solid-phase peptide synthesis.

18. Kit comprising a peptide or polypeptide according to any one of claims 1 to 11, a nucleotide sequence according to claim 12, a gene construct according to claim 13 or a vector according to claim 14.

19. Use of a peptide or polypeptide according to any one of claims 1 to 11, of a nucleotide sequence according to claim 12, of a gene construct according to claim 13, of a vector according to claim 14 or of a kit according to claim 18 :
- for the capture of protein(s) of the ATG8 family and optionally of their interactor(s),
- for the detection of protein(s) of the ATG8 family, or
- for the screening of molecules which inhibit autophagy.
